(19) Europäisches Patentamt
European Patent Office
Office européen des brevets

(11) Veröffentlichungsnummer: **0 003 817**
**B1**

(12) **EUROPÄISCHE PATENTSCHRIFT**

(21) Anmeldenummer: **79100481.5**

(22) Anmeldetag: **19.02.79**

(51) Int. Cl.³: **C 23 F 11/14,**
C 23 F 11/16, C 09 D 5/08

(54) **Korrosionsschutzüberzugsmittel.**

(30) Priorität: **23.02.78 DE 2807698**
**03.06.78 DE 2824508**

(43) Veröffentlichungstag der Anmeldung:
**05.09.79 Patentblatt 79/18**

(45) Bekanntmachung des Hinweises auf die Patenterteilung:
**11.02.81 Patentblatt 81/6**

(84) Benannte Vertragsstaaten:
**FR GB NL SE**

(56) Entgegenhaltungen:
**DE - A - 2 204 985**
**DE - B - 2 502 781**

(73) Patentinhaber: **Henkel Kommanditgesellschaft auf Aktien**
**-Patentabteilung- Postfach 1100 Henkelstrasse 67**
**D-4000 Düsseldorf 1 (DE)**

(72) Erfinder: **Eschwey, Helmut, Dr.**
**Leitenstorffer Strasse 24**
**D-4000 Düsseldorf 13 (DE)**
(72) Erfinder: **Galinke, Joachim, Dr.**
**Falkenweg 6**
**D-4018 Langenfeld (DE)**
(72) Erfinder: **Linden, Heinrich**
**Am Falder 75**
**D-4000 Düsseldorf 13 (DE)**
(72) Erfinder: **Wegemund, Bernd, Dr.**
**Händelweg 3**
**D-5657 Haan (DE)**
(72) Erfinder: **Wiemers, Norbert, Dr.**
**Humboldtstrasse 51**
**D-4019 Monheim-Baumberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 817 B1

# Korrosionsschutzüberzugsmittel

Gegenstand der Erfindung sind Korrosionsschutzüberzugsmittel für Metalloberflächen, insbesondere für Eisen, die neben sonst üblichen Bestandteilen Zink- und/oder Bleisalze organischer Verbindungen als Korrosionsinhibitoren enthalten.

Um Metalle vor Korrosion zu schützen, werden die Metalloberflächen üblicherweise mit Grundierungen oder Lacken beschichtet, die Korrosionsinhibitoren als wirksame Komponente enthalten, um den Korrosionsvorgang zu hemmen oder möglichst vollständig zu unterbinden. Seit langem ist es nun bekannt, als Korrosionsinhibitoren für Korrosionsschutzüberzugsmittel beispielsweise Zinkkaliumchromat, Zinktetrahydroxychromat, Strontiumchromat, Bariumchromat oder auch Bleimennige einzusetzen. In letzter Zeit hat sich jedoch die Aufmerksamkeit der Lackrohstoffhersteller mehr und mehr dem Umweltverhalten derartiger Korrosionsinhibitoren zugewandt, mit dem Ergebnis, daß zum Beispiel so bewährte Inhibitoren wie das Zinkkaliumchromat in zunehmendem Maße durch umweltfreundlichere Korrosionsinhibitoren—wie beispielsweise das Zinkphosphat—ersetzt werden.

Nun beruht die korrosionsinhibierende Wirkung des Zinkphosphats auf der Ausbildung von Schutzschichten in anionischen Bereichen. Elektrochemische Reaktionen wie im Falle des Zinkkaliumchromats treten beim Zinkphosphat nicht auf, so daß bislang stets eine Kombination des Zinkphosphats mit elektrochemisch wirksamen Korrosionsschutzpigmenten empfohlen wurde (Deutsche Farben-Zeitschrift (defazet) *29* (1975) 13—17). Als elektrochemisch wirksame Korrosionsinhibitoren kommen beispielsweise Zink- und/oder Bleisalze der 3-Nitrophthalsäure, 4-Nitrophthalsäure, 5-Nitroisophthalsäure bzw. der Mono-Nitroterephthalsäure—wie sie in den deutschen Auslegeschriften DE - B 22 04 985 und DE - B 25 02 781 beschrieben sind—in Frage. Wenngleich derartige Substanzen zum Teil eine verbesserte korrosionssinhibierende Wirkung (Korrosionsschutzwert) gegenüber herkömmlichen Inhibitoren zeigen, so erweist sich bei ihrem Einsatz in üblichen Bindemittelformulierungen bzw. Lacken, daß die mit solchen Korrosionsinhibitoren versehenen Lacke eine nur geringe Stabilität hinsichtlich des Sedimentationsverhaltens aufweisen. In Lacken, d.h. in Korrosionsschutzüberzugsmitteln, neigen die genannten Substanzen zur schnellen Sedimentation sowie zur Ausbildung eines festen Bodensatzes, wodurch die praktische Verarbeitung derartiger Lacke wesentlich erschwert wird.

Die Aufgabe der vorliegenden Erfindung war es daher, solche Korrosionsinhibitoren für Korrosionsschutzüberzugsmittel bereitzustellen, die einerseits eine möglichst weitgehende Korrosionsinhibierung bewirken sowie andererseits die Sedimentationsstabilität der Mittel nicht negativ beeinflussen und ein gutes Dispergiervermögen aufweisen.

Gegenstand der Erfindung sind somit Korrosionsschutzüberzugsmittel für Metalloberflächen auf Basis üblicher Bestandteile sowie Zink- und/oder Bleisalzen organischer Verbindungen, die dadurch gekennzeichnet sind, daß sie Zink- und/oder Bleisalze von mit mindestens einer Hydroxyl- oder Mercaptogruppe substituierten fünf- oder sechsgliedrigen Heterocyclen, die im Ring mindestens ein Stickstoffatom und konjugierte Doppelbindungen aufweisen, von denen mindestens eine in einer Gruppierung der Formel

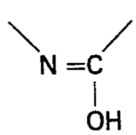 oder 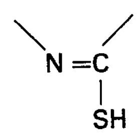

vorliegt enthalten.

Überraschenderweise wurde nämlich gefunden, daß Zink- und/oder Bleisalze der vorstehend näher definierten Heterocyclen in üblichen Bindemittelformulierungen für Korrosionsschutzüberzugsmittel sowohl eine ausgezeichnete korrosionsinhibierende Wirkung zeigen als auch zu stabilen Korrosionsschutzüberzugsmitteln hinsichtlich des Sedimentationsverhaltens führen.

Darüberhinaus weisen derartige Korrosionsinhibitoren gegenüber den aus dem Stand der Technik bekannten elektrochemisch wirksamen Inhibitoren noch weitere Vorteile auf: Die extrem niedrigen Löslichkeitswerte der erfindungsgemäßen Salze in Wasser—die Löslichkeit der Zinksalze in Wasser ist bei 20°C kleiner oder gleich 0,1 %; die der Bleisalze ist kleiner oder gleich 0,01 %—bewirken eine verbesserte Beständigkeit der damit erzeugten Korrosionsschutzüberzüge oder Lackfilme, da diese Salze nicht so leicht aus dem gebildeten Lackfilm ausgewaschen werden können. Die nur geringe Wasserlöslichkeit der erfindungsgemäßen Inhibitoren ist auch hinsichtlich des Umweltverhaltens, d.h. im Hinblick auf eine verminderte Toxizität, positiv zu bewerten.

Beispiele für fünfgliedrige Heterocyclen, die mit Zink und/oder Blei erfindungsgemäße Salze bilden, sind nachstehend angeführt:

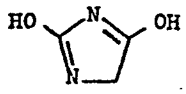

Hydantoin

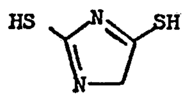

2,4-Dithiohydantoin

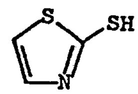

2-Mercaptothiazol

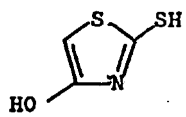

2-Mercapto-4-hydroxythiazol

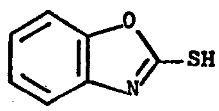

2-Mercaptobenzoxazol

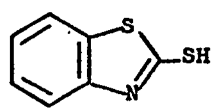

2-Mercaptobenzthiazol

Als Beispiele für entsprechende sechsgliedrige Heterocyclen sind zu nennen:

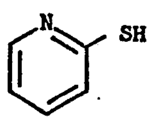

2-Mercaptopyridin

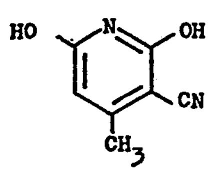

3-Cyan-6-hydroxy-4-methylpyridon-2

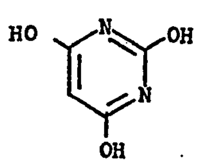

Barbitursäure

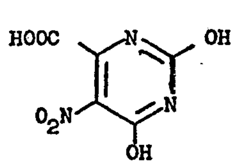

5-Nitroorotsäure

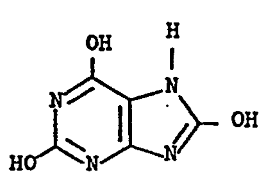

Harnsäure (2,6,8-Trihydroxypurin)

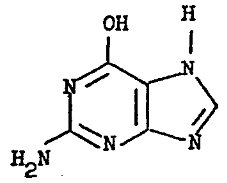

Guanin (2-Amino-6-hydroxypurin)

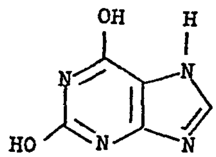

Xanthin (2,6-Dihydroxypurin)

Wie diese Beispiele bereits zeigen, kommen für die erfindungsgemäßen Korrosionsinhibitoren auch solche Heterocyclen in Frage, die zwei Stickstoffatome im Ring oder noch andere Heteroatome aufweisen; ferner sowohl ein- als auch mehrkernige Heterocyclen sowie solche, die neben einer oder mehrerer Hydroxyl- bzw. Mercaptogruppen noch weitere Substituenten enthalten.

Als wirksame Korrosionsinhibitoren im Sinne der Erfindung sind somit beispielsweise die Zink- oder Bleisalze des Pyrrols, Imidazols, Pyrazols, Pyridins, Pyrazins, Pyrimidins, Pyridazins, Isoindols, Indazols, Purins, Chinolins, Isochinolins, Chinazolins, Phenazins, Oxazols, Isooxazols, Thiazols, Benzothiazols, Benzimidazols, Benzoxazols und dergleichen mehr in Betracht zu ziehen, sofern sie mindestens eine Hydroxyl- oder Mercaptogruppe in der im Anspruch 1 näher definierten Gruppierung aufweisen. Einige dieser Verbindungen können auch in der Ketoform vorliegen; zur Salzbildung mit Zink oder Blei ist jedoch nur die entsprechende Enolform geeignet. Für die Mercaptogruppe gilt sinngemäß das gleiche.

Von besonderer Bedeutung im Sinne der Erfindung sind die Zink- oder Bleisalze des Mercaptobenzthiozols sowie des Mercaptopyridins, da mit Korrosionsschutzüberzugsmitteln auf Basis dieser Salze hervorragende Korrosionsschutzwerte erzielt werden und sich auch über längere Zeiträume hinweg kein Bodensatz in derartigen Korrosionsschutzüberzugsmitteln bildet.

Gleichfalls im Sinne der Erfindung bevorzugt sind die Zink- und/oder Bleisalze von ein- oder mehrkernigen s-Triazinderivaten, die mindestens eine Hydroxyl- oder Mercaptogruppe am Triazinring aufweisen.

Beispiele für einkernige s-Triazinderivate, die mit Zink und/oder Blei erfindungsgemäße Salze bilden, sind nachstehend angeführt:
Cyanursäure (2,4,6-Trihydroxy-s-triazin),
Monothiocyanursäure (2,4-Dihydroxy-6-mercapto-s-triazin),
Dithiocyanursäure (2-Hydroxy-4,6-dimercapto-s-triazin),
Trithiocyanursäure (2,4,6-Trimercapto-s-triazin),
Allantoxaidin (2,4-Dihydroxy-s-triazin).

Der Triazinring kann jedoch außer Hydroxyl- oder Mercaptogruppen auch noch weitere Substituenten aufweisen. Entsprechende einkernige Verbindungen sind beispielsweise:
Ammelid (2,4-Dihydroxy-6-amino-s-triazin),
Monothioammelid (2-Hydroxy-4-mercapto-6-amino-s-triazin),
Dithioammelid (2,4-Dimercapto-6-amino-s-triazin),
Ammelin (2-Hydroxy-4,6-diamino-s-triazin),
Thioammelin (2-Mercapto-4,6-diamino-s-triazin),
Acetothioguanid (2-Mercapto-4-amino-6-methyl-s-triazin),
Acetoguanamid (2,4-Dihydroxy-6-methyl-s-triazin),
o-Carboxybenzoguanid (2-Hydroxy-4-amino-6-(o-carboxyphenyl)-s-triazin),
Oxonsäure (2,4-Dihydroxy-6-carboxy-s-triazin),
2-Hydroxy-4,6-dimethoxy-s-triazin,
2,4-Dihydroxy-6-(p-nitrophenyl)-s-triazin,
2,4-Dihydroxy-6-allyloxy-s-triazin.

Neben derartigen einkernigen s-Triazinderivaten kommen ferner auch mehrkernige s-Triazinderivate für die erfindungsgemäßen Zink- und/oder Bleisalze in Betracht, sofern sie pro Triazinring mindestens eine Hydroxyl- oder Mercaptogruppe aufweisen. Hierzu zählen zum Beispiel oligomere Kondensationsprodukte des Ammelins oder des Thioammelins mit Formaldehyd oder aber über Disulfidbrücken gekoppelte Oligomere und Polymere der Thiocyanursäure.

Schließlich fallen auch Zink- und/oder Bleisalze mehrkerniger kondensierter s-Triazinringsysteme mit Hydroxyl und/oder Mercaptogruppen—wie beispielsweise die Cyamelursäure (2,5,8-Trihydroxy-tri-s-triazin)—in den Rahmen der erfindungsgemäßen Korrosionsinhibitoren.

Von besonderer Bedeutung im Sinne der Erfindung sind ferner die Zink- und/oder Bleisalze der Cyanursäure sowie der Thiocyanursäure, da mit Korrosionsschutzüberzugsmitteln auf Basis dieser Salze hervorragende Korrosionsschutzwerte erzielt werden und sich auch über längere Zeiträume hinweg kein Bodensatz in derartigen Korrosionsschutzüberzugsmitteln bildet.

Die Herstellung von Blei- und/oder Zinksalzen der angeführten s-Triazinderivate ist auf verschiedenen Wegen leicht durchführbar und wird nachstehend am Beispiel der Cyanursäure erläutert:

Herstellungsweg 1:

Blei- und/oder Zinkcarbonat wird mit Cyanursäure im kochenden Wasser umgesetzt; unter Kohlendioxid-Entwicklung bilden sich die entsprechenden Metallcyanurate.

Herstellungsweg 2:

Blei- und/oder Zink-nitrat, -chlorid, oder -acetat wird in wäßriger Lösung bei Raumtemperatur mit einer wäßrigen Lösung des Trinatrium- oder Trikaliumsalzes der Cyanursäure versetzt; die entsprechenden Metallcyanurate fallen aus der wäßrigen Lösung aus.

Herstellungsweg 3:

Zink- und/oder Bleioxid wird mit Cyanursäure in kochendem Wasser umgesetzt, wobei vorteilhafterweise ein geringer Zusatz von Essigsäure als Katalysator dient; es bilden sich die entsprechenden Metallcyanurate.

Die Herstellung weiterer Zink- oder Bleisalze wird nachstehend am Beispiel des Mercaptobenzthiazols erläutert:

Herstellungsweg 1:

Blei- oder Zinkcarbonat wird mit Mercaptobenzthiazol in kochendem Wasser umgesetzt; unter Kohlendioxid-Entwicklung bilden sich die entsprechenden Metallsalze des Mercaptobenzthiazols.

Herstellungsweg 2:

Blei- oder Zink-nitrat, -chlorid oder -acetat wird in wäßriger Lösung bei Raumtemperatur mit einer wäßrigen Lösung des Natrium- oder Kaliumsalzes des Mercaptobenzthiazols versetzt; die entsprechenden Metallsalze des Mercaptobenzthiazols fallen aus der wäßrigen Lösung aus.

Herstellungsweg 3:

Zink- oder Bleioxid wird mit Mercaptobenzthiazol in kochendem Wasser umgesetzt, wobei vorteilhafterweise ein geringer Zusatz von Essigsäure als Katalysator dient; es bilden sich die entsprechenden Metallsalze des Mercaptobenzthiazols.

Die erfindungsgemäßen Salze lassen sich gut in die für Korrosionsschutzüberzugsmittel üblichen Bindemittelformulierungen durch Einrühren einarbeiten. Im allgemeine reichen schon geringe Mengen dieser Salze zur Erzielung der beschriebenen Effekte aus; d.h. die Salze werden in Mengen von 0,5 bis 10 Gew.-%. vorzugsweise 1 bis 3 Gew.-% bezogen auf die Gesamtmischung, derartigen Bindemittelformulierungen zugesetzt. Als Bindemittel für derartige Lacke kommen die für solche Zwecke gebräuchlichen Harze in Betracht, die in geeigneten Lösungsmitteln gelöst und mit den hierfür bekannten Pigmenten und Füllstoffen versetzt werden.

Die nachstehenden Beispiele zeigen die Wirksamkeit der erfindungsgemäßen Korrosionsschutzüberzugsmittel, sowohl im Hinblick auf die hiermit erzielten Korrosionsschutzwerte (KW) als auch im Hinblick auf das verbesserte Absetzverhalten derartiger Lacke. Zum Vergleich wurden den erfindungsgemäßen Mitteln solche gegenübergestellt, die als Korrosionsinhibitoren Zink- und Zink/Bleisalze der Nitroisophthalsäure sowie ferner Zinkkaliumchromat und Zinkphosphat alleine enthielten.

Zur Ermittlung der in den Beispielen Wiedergegebenen Werte wurden die nachfolgend geschilderten Prüfmethoden angewandt:

A. Korrosionsverhalten

Korrosionsschutzüberzugsmittel gemäß der im jeweiligen Beispiel angeführten Rezeptur wurden mit Hilfe eines Lackschicht-Schleudergerätes (typ 334/II der Firma Erichsen, Bundesrepublik Deutschland) auf rostfreie und entfettete Stahlbleche der Größe 150 x 70 x 1 mm in der Weise aufgetragen, daß Trockenfilmstärken im Bereich von 30 bis 33 $\mu$m resultierten. Nach erfolgter Trocknung der gebildeten Lackfilme—2 Stunden bei 60° C sowie 8 Tage bei Raumtemperatur—wurden für jeden getesteten Korrosionsinhibitor jeweils zwei mit Korrosionsschutzüberzugsmittel beschichtete Stahlbleche dem Salzsprühtest gemäß ASTM B 117—64 (Dauersprühbelastung mit einer 5% igen Kochsalzlösung bei 35°C) für die Dauer von 200 Stunden unterworfen.

Eins der Prüfbleche blieb unverletzt, das zweite wurde jeweils vor dem Salzsprühtest mit einem sogenannten Andreaskreuz versehen (Andreaskreuz = Kreuzschnitt mit einer Klinge von 0,1 mm durch den Lackfilm bis auf den Untergrund),

Zwei weitere beschichtete Stahlbleche—jeweils pro getestem Korrosionsinhibitor—wurden nach DIN 50 018 ohne Andreaskreuz dem Kesternich-Test für die Dauer von 12 Zyklen (1 Zyklus = 8 Stunden bei 40° C in einer feuchten Atmosphäre, die 0,2 l Schwefeldioxid enthält; Entlüften und weitere 16 Stunden ohne Belastung) unterworfen.

Die Beurteilung der belasteten Prüfbleche erfolgte nach einer Rostgradskala gemäß DIN 53 210. Entsprechend der von Ruf angegebenen Methode (Farbe und Lack *75* (1969) 943—949) wurde aus den gewonnenen Einzelergebnissen der sogenannte Korrosionsschutzwert (KW) errechnet.

B. Absetzverhalten

Zur Untersuchung des Absetzverhaltens der Pigmente bzw. Korrosionsinhibitoren wurden 250 ml-Weithalsglasflaschen mit Proben der in den Beispielen beschriebenen Korrosionsschutzüberzugsmittel gefüllt. Durch vorsichtiges manuelles Abtasten mit einem 3 mm breiten Stahlspachtel wurde jeweils nach bestimmten Zeitabständen die Struktur des vorliegenden Bodensatzes ermittelt. Die Beurteilung der Struktur des Bodensatzes erfolgte hierbei nach einer Bewertungsskala mit Kennzahlen von 0 bis 4. Dieser Bewertungsskala lag folgender Schlüssel zugrunde:

| Kennzahl | Bodensatz | Ist das Pigment aufrührbar? | Ist der Lack für die Praxis brauchbar? |
|---|---|---|---|
| 0 | kein | — | ja |
| 1 | leichter | leicht | ja |
| 2 | mäßiger | mäßig | bedingt |
| 3 | starker | schwer | kaum |
| 4 | sehr starker | nein, zementiert | nein |

Beispiel 1

Zusammensetzung der verwendeten Bindemittelformulierung:

34,0 Gewichtsteile kurzöliges, harzmodifiziertes Alkydharz mit 44% Leinöl/Holzöl, 60%ig in Xylol
9,0 Gewichtsteile Xylol
5,6 Gewichtsteile Benzin, Kp: 145 bis 200°C
2,3 Gewichtsteile Äthylglykol
2,3 Gewichtsteile Dekalin
0,2 Gewichtsteile Calciumnaphthenat, 4% Ca
0,4 Gewichtsteile Kobaltnaphthenat, 6% Co
0,1 Gewichtsteile Bleinaphthenat, 24% Pb
0,3 Gewichtsteile Methyläthylketoxim
6,8 Gewichtsteile Titandioxid, Rutil
4,5 Gewichtsteile Mikrotalkum
23,7 Gewichtsteile Schwerspat
8,5 Gewichtsteile Zinkphosphat

---

97,7 Gewichtsteile Lack

Diesem Lack wurden jeweils 2,3 Gewichtsteile der nachfolgend genannten Korrosionsinhibitoren zugesetzt; die Beispiele 1.1 bis 1.12 betreffen erfindungsgemäße Salze, die Beispiele 1.13 bis 1.16 Inhibitoren gemäß dem Stand der Technik, wobei in Beispiel 1.16 Zinkphosphat alleine ohne weiteren Inhibitorzusatz vorlag.

| Beispiel | Korrosionsinhibitor | KW in % | Bodensatz nach 3 Tagen | 21 Tagen |
|---|---|---|---|---|
| 1. 1 | Bleisalz des Mercaptopyridins (44,0 % Pb) | 95 | 0 | 0 |
| 1. 2 | Zinksalz des Mercaptopyridins (22,6 % Zn) | 89 | 0 | 0 |
| 1. 3 | Bleisalz des 3-Cyan-6-hydroxy-4-methylpyridon-2 (56,4 % Pb) | 88 | 0 | 0 |
| 1. 4 | Zinksalz des 3-Cyan-6-hydroxy-4-methylpyridon-2 (30,2 % Zn) | 80 | 0 | 0 |
| 1. 5 | Bleisalz der Barbitursäure (64,5 % Pb) | 88 | 0 | 0 |
| 1. 6 | Zinksalz der Barbitursäure (35,2 % Zn) | 78 | 0 | 0 |
| 1. 7 | Bleisalz der 5-Nitroorotsäure (57,9 % Pb) | 90 | 0 | 0 |
| 1. 8 | Zinksalz der 5-Nitroorotsäure (28,8 % Zn) | 81 | 0 | 0 |
| 1. 9 | Bleisalz des Hydantoins (56,9 % Pb) | 83 | 0 | 0 |
| 1.10 | Zinksalz des Hydantoins (40,0 % Zn) | 75 | 0 | 0 |
| 1.11 | Bleisalz des Mercaptobenzthiazols (37,3 % Pb) | 96 | 0 | 0 |
| 1.12 | Zinksalz des Mercaptobenzthiazols (16,4 % Zn) | 88 | 0 | 0 |
| 1.13 | 5-Nitroisophthalsaures Zink (44,1 % Zn) | 73 | 2 | 4 |
| 1.14 | 5-Nitroisophthalsaures Zink/Blei (31,0 % Zn/19,1 % Pb) | 83 | 1 | 2 |
| 1.15 | Zinkkaliumchromat bleihaltig nach DIN 55902 | 70 | 0 | 0 |
| 1.16 | Schwerspat (d.h. Zinkphosphat alleine) | 55 | 0 | 0 |

(KW = Korrosionsschutzwert).

Die PVK-Werte (Pigmentvolumenkonzentration) der Korrosionsschutzüberzugsmittel der Beispiele 1 bis 16 lagen im Bereich von 39,4% bis 39,8%.

Die angeführten Beispiele zeigen deutlich die ausgezeichnete korrosionsinhibierende Wirksamkeit der Salze des Mercaptopyridins sowie des Mercaptobenzthiazols. Hervorzuheben sind hierbei insbesondere die Salze des Mercaptobenzthiazols, da diese bei einem bemerkenswert geringen Blei- bzw. Zinkgehalt eine hervorragende Wirksamkeit aufweisen. So enthält ein Lack mit beispielsweise 2,3 Gew.-% Mercaptobenzthiazolsalz im Falle des Bleisalszes (37,3% Pb) nur 0,86% Blei und im Falle des Zinksalzes (16,4% Zn) nur 0,38% Zink; wobei der Blei- bzw. Zinkanteil der übrigen Komponenten der Bindemittelformulierung gesondert berücksichtigt werden muß.

Beispiel 2

Zusammensetzung der verwendeten Bindemittelformulierung:

34,0 Gewichtsteile kurzöliges, harzmodifiziertes Alkydharz mit 44% Leinöl/Holzöl, 60%ig in Xylol
9,0 Gewichtsteile Xylol
5,6 Gewichtsteile Benzin, Kp: 145 bis 200°C
2,3-Gewichtsteile Äthylglykol
2,3 Gewichtsteile Dekalin
0,2 Gewichtsteile Calciumnaphthenat, 4% Ca
0,4 Gewichtsteile Kobaltnaphthenat, 6% Co
0,1 Gewichtsteile Bleinaphthenat, 24% Pb
0,3 Gewichtsteile Methyläthylketoxim
6,8 Gewichtsteile Titandioxid, Rutil
4,5 Gewichtsteile Mikrotalkum
23,7 Gewichtsteile Schwerspat
8,5 Gewichtsteile Zinkphosphat

---

97,7 Gewichtsteile Lack

Diesem Lack wurden jeweils 2,3 Gewichtsteile der nachfolgend genannten Korrosionsinhibitoren zugesetzt; die Beispiele 2,1 bis 2,7 betreffen erfindungsgemäße Salze, 2,8 bis 2,11 Inhibitoren gemäß dem Stand der Technik, wobei in Beispiel 2,11 Zinkphosphat alleine ohne weiteren Inhibitorzusatz vorlag.

| Beispiel | Korrosionsinhibitor | KW in % | Bodensatz nach | |
|---|---|---|---|---|
| | | | 3 Tagen | 21 Tagen |
| 2, 1 | Zinktrithiocyanurat (34,2 % Zn) | 83 | 0 | 0 |
| 2, 2 | Bleitrithiocyanurat (55,6 % Pb) | 90 | 0 | 0 |
| 2, 3 | Zink-Blei-Cyanurat (61,2 % Pb; 3,9% Zn) | 85 | 0 | 0 |
| 2, 4 | Bleicyanurat (66,1 % Pb) | 90 | 0 | 0 |
| 2, 5 | Zinkcyanurat (31,7 % Zn) | 80 | 0 | 0 |
| 2, 6 | Thioammelin-Bleisalz (41,2 % Pb) | 78 | 0 | 0 |
| 2, 7 | Thioammelin-Zinksalz (18,9 % Zn) | 73 | 0 | 0 |
| 2, 8 | 5-Nitroisophthalsaures Zink (44,1 % Zn) | 73 | 2 | 4 |
| 2, 9 | 5-Nitroisophthalsaures Zink/Blei (31,0 % Zn; 19,1 % Pb) | 83 | 1 | 2 |
| 2,10 | Zinkkaliumchromat bleihaltig nach DIN 55 902 | 70 | 0 | 0 |
| 2,11 | Schwerspat (d.h. Zinkphosphat alleine) | 55 | 0 | 0 |

(KW = Korrosionsschutzwert).

### Beispiel 3

Es wurde eine dem Beispiel 1 analoge Bindemittelformulierung, jedoch ohne einen Zusatz von Zinkphosphat, verwendet; Zusammensetzung:

34,1 Gewichtsteile kurzöliges, harzmodifiziertes Alkydharz mit 44% Leinöl/Holzöl, 60%ig in Xylol
9,1 Gewichtsteile Xylol
5,7 Gewichtsteile Benzin, Kp: 145 bis 200°C
2,3 Gewichtsteile Äthylglykol
2,3 Gewichtsteile Dekalin
0,2 Gewichtsteile Calciumnaphthenat, 4% Ca
0,4 Gewichtsteile Kobaltnaphthenat, 6% Co
0,1 Gewichtsteile Bleinaphthenat, 24% Pb
0,3 Gewichtsteile Methyläthylketoxim
9,1 Gewichtsteile Titandioxid, Rutil
9,1 Gewichtsteile Mikrotalkum
25,0 Gewichtsteile Schwerspat

---

97,7 Gewichtsteile Lack

Diesem Lack wurden gleichfalls jeweils 2,3 Gewichtsteile der nachfolgend genannten Korrosionsinhibitoren zugesetzt; die Beispiele 3,1 und 3,2 betreffen erfindungsgemäße Salze, 3,3 und 3,4 Vergleichsverbindungen.

| Beispiel | Korrosionsinhibitor | KW in % | Bodensatz nach | |
|---|---|---|---|---|
| | | | 3 Tagen | 21 Tagen |
| 3, 1 | Bleicyanurat (66,1 % Pb) | 83 | 0 | 0 |
| 3, 2 | Zinkcyanurat (31,7 % Zn) | 73 | 0 | 0 |
| 3, 3 | 5-Nitroisophthalsaures Zink/Blei (31,0 % Zn 19,1 % Pb) | 75 | 1 | 2 |
| 3, 4 | Zinkkaliumchromat, bleihaltig nach DIN 55 902 | 50 | 0 | 0 |

Die PVK-Werte (Pigmentvolumenkonzentration) der Korrosionsschutzüberzugsmittel der Beispiele 2 und 3 lagen im Bereich von 39,4 bis 39,8%.

Der mit den erfindungsgemäßen Korrosionsschutzüberzugsmitteln verbundene Effekt eines verbesserten Absetzverhaltens wird in den nachfolgenden Beispielen an zwei weiteren Bindemittelformulierungen aufgezeigt, wobei die Antiabsetzwirkung der s-Triazinsalze nach der Erfindung deutlich in Erscheinung tritt.

Beispiel 4

Zusammensetzung der verwendeten Einbrenngrundierung auf Basis eines Epoxyharzesters:

400 Gewichtsteile Epoxyharz-Ricinensäureester mit einem Fettsäuregehalt von 42%, 60%ig in Xylol
110 Gewichtsteile Zinkphosphat
120 Gewichtsteile Mikrotalkum
80 Gewichtsteile Titandioxid, Rutil
193,5 Gewichtsteile Schwerspat
15 Gewichtsteile Äthylglykol
15 Gewichsteile n-Butanol
30 Gewichtsteile Tetralin
110 Gewichsteile höhere Aromaten
110 Gewichtsteile nich plastifiziertes Harnstoffharz, 65%ig in n-Butanol
200 Gewichtsteile Xylol

1373,5 Gewichtsteile Lack

Diesem Lack wurden jeweils 26,5 Gewichtsteile der nachfolgend genannten Korrosionsinhibitoren zugesetzt; wobei in Beispiel 4,4 Zinkphosphat ohne weiteren Inhibitorzusatz vorlag. Die Pigmentvolumenkonzentration (PVK) betrug ca. 34%.

| Beispiel | Korrosionsinhibitor | Bodensatz nach Tagen | | | | | |
|---|---|---|---|---|---|---|---|
| | | 3 | 7 | 14 | 21 | 30 | 40 |
| 4, 1 | Zinkcyanurat (31,7 % Zn) | 0 | 0 | 0 | 0 | 0 | 0 |
| 4, 2 | Nitroisophthalsaures Zink (44,1 % Zn) | 3–4 | 4 | 4 | 4 | 4 | 4 |
| 4, 3 | Nitroisophthalsaures Zink /Blei (31,0 % Zn; 19.1 % Pb) | 3–4 | 4 | 4 | 4 | 4 | 4 |
| 4, 4 | Schwerspat, d.h. Zinkphosphat alleine | 0–1 | 2 | 3–4 | 4 | 4 | 4 |

Beispiel 5

Zusammensetzung der verwendeten lufttrocknenden Grundierung:

370 Gewichtsteile Leinölalkyd mit einem Ölgehalt von 38% und mit 20% Harz modifiziert 60%ig in Xylol
80 Gewichtsteile Zinkphosphat
110 Gewichtsteile Mikrotalkum
168 Gewichtsteile Schwerspat
60 Gewichtsteile Titandioxid, Rutil
20 Gewichtsteile Dekalin
15 Gewichtsteile Äthvlalvkol
1 Gewichtsteil Kobaltnaphthenat, 6% Co
4 Gewichtsteile Bleinaphthenat, 24% Pb
1 Gewichtsteil Mangannaphthenat, 6% Mn
56 Gewichtsteile höhere Aromaten
3 Gewichtsteile Methyläthylketoxim
190 Gewichtsteile Xylol

---

1078 Gewichtsteile Lack

Diesem lack wurden jeweils 22 Gewichtsteile der nachfolgend genannten Korrosionsinhibitoren zugesetzt; wobei in Beispiel 5,3 Zinkphosphat ohne weiteren Inhibitorzusatz vorlag. Die Pigmentvolumenkonzentration (PVK) betrug ca. 38%

| Beispiel | Korrosionsinhibitor | Bodensatz nach Tagen | | | | | |
|---|---|---|---|---|---|---|---|
| | | 3 | 7 | 14 | 21 | 30 | 40 |
| 5, 1 | Zinkcyanurat (31,7 % Zn) | 0 | 0 | 0 | 0 | 0 | 0–1 |
| 5, 2 | 5-Nitroisophthalsaures Zink (44,1 % Zn) | 1 | 1–2 | 2 | 2 | 2–3 | 3–4 |
| 5, 3 | Schwerspat, d.h. Zinkphosphat alleine | 0 | 0 | 0 | 0 | 0 | 0–1 |

Beispiel 6

Im Hinblick auf das durch die erfindungsgemäßen Salze bedingte, erwiesenermaßen verbesserte Absetzverhalten der Korrosionsschutzüberzugsmittel wurde der mittlere Teilchendurchmesser $\bar{d}$ einiger der verwendeten Korrosionsinhibitoren mit Hilfe des sogenannten Coulter-Counter-Gerätes besitmmt. Dieses Gerät arbeitet nach einem Verfahren, bei dem eine verdünnte und elektrisch leitende Suspension durch eine Blende (20 bis 400 µm) strömt. Beim Durchtritt eines Teilchens durch diese Blende ändert sich die elektrische Leitfähigkeit proportional zum Volumen des Teilchens. Die so erhaltenen Impulse werden gezählt und bezüglich ihrer Höhe analysiert (vgl. H. Kittel, Lehrbuch der Lacke und Beschichtungen, Band II, Colomb, Berlin 1974, Seite 512 sowie T. C. Patton, Pigment Handbook, Volume III, Wiley, New York 1973, Seite 101—106). Die folgenden Werte wurden ermittelt:

| Korrosionsinhibitor | Mittlerer Teilchendurchmesser $\bar{d}$ in µm |
|---|---|
| Zinkcyanurat (31,7 % Zn) | 10,0 |
| Zink-Mercaptobenzthiazol (16,4 % Zn) | 9,5 |
| 5-Nitroisophthalsaures Zink (44,1 % Zn) | 5,0 |
| 5-Nitroisophthalsaures Zink/Blei (31,0 % Zn; 19,1 % Pb) | 8,5 |

Die überraschend gute Antiabsetzwirkung des Zinkcyanurats sowie des Zink-Mercaptobenzthiazols ist mithin nicht durch diue Teilchengröße bedingt, da alle ermittelten Werte für d in der gleichen Größenordnung liegen.

**Patentansprüche:**

1. Korrosionsschutzüberzugsmittel für Metalloberflächen auf Basis üblicher Bestandteile sowie Zink- und/oder Bleisalzen organischer Verbindungen, die dadurch gekennzeichnet, daß sie Zink- und/oder Bleisalze von mit mindestens einer Hydroxyl- oder Mercaptogruppe substituierten fünf- oder sechsgliedrigen Heterocyclen, die im Ring mindestens ein Stickstoffatom und konjugierte Doppelbindungen aufweisen, von denen mindestens eine in einer Gruppierung der Formel

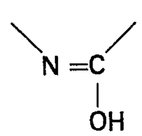

oder

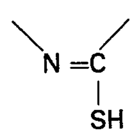

vorliegt enthalten.

2. Korrosionsschutzüberzugsmittel nach Anspruch 1, dadurch gekennzeichnet, daß sie Zink- oder Bleisalze des Mercaptobenzthiazols enthalten.

3. Korrosionsschutzüberzugsmittel nach Anspruch 1, dadurch gekennzeichnet, daß sie Zink- oder Bleisalze des Mercaptopyridins enthalten.

4. Korrosionsschutzüberzugsmittel nach Anspruch 1, dadurch gekennzeichnet, daß die Zink- und/oder Bleisalze von ein- oder mehrkernigen s-Triazinderivaten, die mindestens eine Hydroxyl- oder Mercaptogruppe am Triazinring aufweisen, enthalten.

5. Korrosionsschutzüberzugsmittel nach Anspruch 4, dadurch gekennzeichnet, daß sie Zink- und/oder Bleisalze der Cyanursäure enthalten.

6. Korrosionsschutzüberzugsmittel nach Anspruch 4, dadurch gekennzeichnet, daß sie Zink- und/oder Bleisalze der Thiocyanursäure enthalten.

**Revendications**

1. Agents de revêtement de protection contre la corrosion pour les surfaces métalliques à base des constituants habituels, ainsi que de sels de zinc et/ou de plomb de substances organiques, caractérisés en ce qu'ils contiennent des sels de zinc et/ou de plomb d'hétérocycles à 5 ou 6 chaînons

substitués par au moins un groupe hydroxy ou mercapto, qui présentent dans le noyau au moins un atome d'azote et des doubles liaisons conjuguées dont l'une au moins fait partie d'un groupement de formule

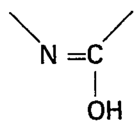

ou

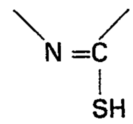

2. Agents de revêtement de protection contre la corrosion selon la revendication 1, caractérisés en ce qu'ils contiennent des sels de zinc ou de plomb du mercaptobenzothiazole.

3. Agents de revêtement de protection contre la corrosion selon la revendication 1, caractérisés en ce qu'ils contiennent des sels de zinc ou de plomb de mercaptopyridine.

4. Agents de revêtement selon la revendication 1, caractérisés en ce qu'ils contiennent des sels de zinc et/ou de plomb de dérivés mono- ou polynucléaires de s-triazine, qui présentent au moins un groupe hydroxy ou mercapto dans le noyau triazine.

5. Agents de revêtement de protection contre la corrosion selon la revendication 4, caractérisés en ce qu'ils contiennent des sels de zinc et/ou de plomb de l'acide cyanurique.

6. Agents de revêtement de protection contre la corrosion selon la revendication 4, caractérisés en ce qu'ils contiennent des sels de zinc et/ou de plomb de l'acide thiocyanurique.

**Claims**

1. Anti-corrosion coating compositions for metal surfaces based on the usual constituents in conjunction with zinc and/or lead salts of organic compounds, characterised in that they contain zinc and/or lead salts of five- or six-membered heterocycles which are substituted by at least one hydroxyl or mercapto group and which contain in the ring at least one nitrogen atom and conjugated double bonds of which at least one is present in a group corresponding to the formula

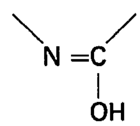

or

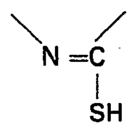

2. Anti-corrosion coating compositions as claimed in Claim 1, characterised in that they contain zinc or lead salts of mercaptobenzthiazole.

3. Anti-corrosion coating compositions as claimed in Claim 1, characterised in that they contain zinc or lead salts of mercaptopyridine.

4. Anti-corrosion coating compositions as claimed in Claim 1, characterised in that they contain zinc and/or lead salts of mononuclear or polynuclear *s*-triazine derivatives containing at least one hydroxyl or mercapto group on the triazine ring.

5. Anti-corrosion coating compositions as claimed in Claim 4, characterised in that they contain zinc and/or lead salts of cyanuric acid.

6. Anti-corrosion coating compositions as claimed in Claim 4, characterised in that they contain zinc and/or lead salts of thiocyanuric acid.